# EUROPÄISCHE PATENTSCHRIFT

(11) **EP 2 646 591 B1**
(45) Veröffentlichungstag und Bekanntmachung des Hinweises auf die Patenterteilung: **19.06.2019**
(21) Anmeldenummer: 11810543.6
(22) Anmeldetag: 16.11.2011
(51) Int. Cl.: C22F 1/18, A61L 27/06, B21C 23/00, C22C 14/00, B21C 23/18, B21K 1/46

(54) **VERFAHREN ZUR HERSTELLUNG EINES GEGENSTANDES AUS EINEM METALL ODER EINER LEGIERUNG MITTELS STARKER PLASTISCHEN VERFORMUNG SOWIE PRESSWERKZEUG HIERFÜR**
METHOD FOR PRODUCING AN OBJECT FROM A METAL OR AN ALLOY BY MEANS OF LARGE PLASTIC DEFORMATION AND PRESSING TOOL THEREFOR
PROCÉDÉ DE PRODUCTION D'UN OBJET EN MÉTAL OU EN ALLIAGE PAR DÉFORMATION PLASTIQUE SÉVÈRE ET OUTIL DE MOULAGE PAR COMPRESSION CORRESPONDANT

(30) Priorität: 29.11.2010 AT 19842010
(43) Veröffentlichungstag der Anmeldung: 09.10.2013
(73) Patentinhaber: AIT Austrian Institute of Technology GmbH, 1220 Wien (AT)
(72) Erfinder: KRYSTIAN, Maciej, A-1210 Wien (AT); MINGLER, Bernhard, A-1110 Wien (AT)
(74) Vertreter: Wirnsberger, Gernot
(86) Internationale Anmeldenummer: PCT/AT2011/050030
(87) Internationale Veröffentlichungsnummer: WO 2012/071600

(56) Entgegenhaltungen:
- WO-A2-2010/047620
- WO-A2-2010/074438
- JP-A- 2002 248 517
- US-B1- 6 399 215
- XU W ET AL: "Formation of an ultrafine-grained structure during equal-channel angular pressing of a beta-titanium alloy with low phase stability", SCRIPTA MATERIALIA, ELSEVIER, AMSTERDAM, NL, vol. 60, no. 11, 26 February 2009 (2009-02-26), pages 1012-1015, XP026075509, ISSN: 1359-6462, DOI: 10.1016/J.SCRIPTAMAT.2009.02.043 [retrieved on 2009-02-26]
- TAIK NAM KIM ET AL: "In vitro biocompatibility of equal channel angular processed (ECAP) titanium; In vitro biocompatibility of ECAP titanium", BIOMEDICAL MATERIALS, INSTITUTE OF PHYSICS PUBLISHING, BRISTOL, GB, vol. 2, no. 3, 1 September 2007 (2007-09-01), pages S117-S120, XP020125634, ISSN: 1748-605X, DOI: 10.1088/1748-6041/2/3/S06

## Beschreibung

Die Erfindung betrifft ein Verfahren zur Herstellung eines Gegenstandes aus einem Metall oder einer Legierung, insbesondere einer Titanlegierung, wobei in einem Pressvorgang ein Bolzen gegebenenfalls mehrmals wiederholt durch einen ersten Kanal und einen daran anschließenden zweiten Kanal eines Presswerkzeuges gepresst wird, wonach aus dem gepressten Bolzen der Gegenstand wie ein Implantat erstellt wird.

Schließlich betrifft die Erfindung ein Presswerkzeug zum Umformen eines Bolzens, aufweisend einen ersten Kanal und einen daran anschließenden zweiten Kanal, wobei der erste Kanal und der zweite Kanal einen von 0° verschiedenen Winkel miteinander einschließen.

Aus dem Stand der Technik ist ein sogenanntes Equal-Channel-Angular-Pressing-Verfahren (ECAP-Verfahren) zum Umformen von metallischen oder aus einer Legierung bestehenden Werkstücken oder Halbzeugen bekannt. Bei diesem Verfahren handelt es sich um eine Variante von Verfahren, die in der Fachsprache als "severe plastic deformation" (SPD) bezeichnet werden. Diesen Verfahren ist gemein, dass eine massive Umformung des eingesetzten Werkstückes bzw. Halbzeuges erfolgt, insbesondere um mechanische Kennwerte zu steigern. Bei einem ECAP-Verfahren können in besonderer Weise feine Gefüge und damit hohe mechanische Kennwerte eines Metalls oder einer Legierung erreicht werden. Eine vorgenommene Umformung, die durch mehrmaliges Pressen eines Bolzens unter erhöhtem hydrostatischen Druck durch einen ersten Kanal und einen dazu angewinkelt, d. h. in einem Winkel von mehr als 0° bis weniger als 180°, verlaufenden, zweiten Kanal erfolgt, ergibt im Ergebnis eine Massivumformung, die zu einem gewünschten feinen Gefüge, in der Regel im Submikrometerbereich, teilweise auch Nanometerbereich, und hohen mechanischen Kennwerten des gepressten Bolzens führt. Verschiedene Verfahrensvarianten eines ECAP-Verfahrens sind in R. Z. Valiev et al., Prog. Mat. Sci. 51, 2006, 881 offenbart.

Aus dem Stand der Technik ist es auch bekannt, Titan oder Titanlegierungen, insbesondere eine Titanlegierung mit 6 Masse-% Aluminium und 4 Masse-% Vanadium, Titan als Rest neben herstellungsbedingten Verunreinigungen und Begleitelementen (in der Folge kurz: Ti64-Legierung) als Halbzeug für Teile für die Raum- und Luftfahrt und eine besonders reine Variante dieser Legierung (in der Folge kurz: Ti64ELI-Legierung) für Halbzeug, z. B. Bolzen, zur Herstellung von Medizinprodukten wie Implantaten einzusetzen. Die Implantate oder andere Teile werden aus einem Halbzeug durch Schmieden oder spanabhebende Bearbeitung in eine für die jeweilige Anwendung erforderliche Geometrie gebracht.

Bekannt ist es aus der Primärliteratur auch, dass ein ECAP-Verfahren für Ti64ELI-Legierungen angewendet wird, um mechanische Eigenschaften entsprechend behandelter Legierungen im Hinblick auf einen Einsatz für Implantate zu untersuchen (R. Z. Valiev et al., Rev. Adv. Mater. Sci. 25, 2010, 1; L. R. Saitova et al., Int. J. Fat. 31, 2009, 322; L. R. Saitova et al., Mat. Sci. Eng. A503, 2009, 145). Allerdings sollen gemäß der US 6,399,215 B1 derartige Legierungen für Implantate nicht besonders geeignet sein, da diese durch Verschleiß einzelne Elemente in einen Körper abgeben und Vanadium und Aluminium potenziell giftig sein sollen.

Bei einem ECAP-Verfahren wird in der Regel zwar ein weitgehend homogenes Gefüge eines gepressten Bolzens erhalten, man ist aber bestrebt, ein solches Verfahren in Bezug auf eine Minimierung von erforderlichen Pressvorgängen und eine dabei erreichte Sättigung der mechanischen Kennwerte auf hohem Niveau und bei hoher Gefügehomogenität weiter zu verbessern. Hierfür wird eine in der Fachsprache als "backpressure" bezeichnete Maßnahme eingesetzt, um eine Art Gegendruck zu erzeugen bzw. einen hydrostatischen Druck im Werkzeug, insbesondere in einem Bereich eines am Übergang der Kanäle gebildeten Knickes, wo die plastische Verformung stattfindet, zu erhöhen. Ein derartiger Gegendruck kann beispielsweise mechanisch durch einen Kolben oder durch eine hydraulische Flüssigkeit aufgebracht werden. Durch den Gegendruck sind die ECAP- bzw. SPD-Verformungsbedingungen noch massiver, sodass ein Bolzen gegebenenfalls mit weniger Pressvorgängen auf ein gewünschtes Eigenschaftsprofil hin umgeformt werden kann. Nachteilig dabei ist allerdings ein hoher apparativer Aufwand.

In der JP 2002 248517 A ist ein gattungsgemäßes Werkzeug zum Umformen eines thermoelektrischen Materials offenbart.

In der WO 2010/074438 A2 ist ein Verfahren zum kontinuierlichen Ziehen eines Drahtes offenbart, wobei ein Presswerkzeug eingesetzt wird, das zwei in einem von 0° verschiedenen Winkel angeordnete Kanäle aufweist, wobei der zweite Kanal verjüngt ist.

In der US 6,399,215 B1 und in der WO 2010/047620 A2 ist offenbart, dass Werkstücke aus Titan für biomedizinische Zwecke durch ECAP mit einem besonders feinen Gefüge hergestellt werden können.

In dem Dokument T.N. Kim et al., In vitro biocompatibility of equal channel angular processed (ECAP) titanium, Biomed. Mater. 2, 2007, S117 wird die Biokompatibilität von reinem Titan und einer Legierung des Typs Ti6Al4V, die mittels ECAP verformt sind, unter anderem in Bezug auf die Korngröße in den Materialien diskutiert.

In dem Dokument W. Xu et al., Formation of an ultrafine-grained structure during equalchannel angular pressing of β-titanium alloy with low phase stability, Scripta Materalia 60, 2009, 1012, ist die Bildung einer ultrafeinen Kornstruktur in einer Titanlegierung offenbart, die mittels ECAP umgeformt wurde.

Aufgabe der Erfindung ist es, ein Verfahren der eingangs genannten Art anzugeben, mit dem sich auf einfache Weise und ohne große apparative Aufwände eine hohe Gefügehomogenität eines gepressten Bolzens erzielen lässt.

Des Weiteren ist es eine Aufgabe der Erfindung, ein hierfür geeignetes Presswerkzeug anzugeben.

Die verfahrensmäßige Aufgabe wird erfindungsgemäß mit einem Verfahren nach Anspruch 1 gelöst.

Ein mit der Erfindung erzielter Vorteil ist insbesondere darin zu sehen, dass ein in das Presswerkzeug eingeführter und in diesem gepresster Bolzen unter erhöhtem hydrostatischen Druck verformt wird, wobei der Druck innerhalb bestimmter Grenzen gezielt steuerbar ist. Dies wird durch den zumindest an einer Stelle kleineren Durchmesser des zweiten Kanals auf einfache Weise erreicht, ohne dass aufwendige zusätzliche mechanische Komponenten zur Erzeugung eines Gegendrucks erforderlich sind. Durch die entsprechende Druckbelastung bzw. eine auf den Bolzen beim Pressen einwirkende Kraft kann eine hohe Gefügehomogenität erreicht werden. Aufgrund der vorgesehenen Änderung des freien Durchmessers im Bereich des zweiten Kanals ist zwar eine höhere Presskraft erforderlich, die allerdings ohne zusätzliche Modifikationen von in diesem Zusammenhang eingesetzten Presswerkzeugen erreichbar ist.

Bevorzugt ist es, dass der Bolzen durch einen von einer ersten Stelle in Richtung zu einem Auslass hin zumindest teilweise verjüngten, vorzugsweise konisch verjüngten, zweiten Kanal gepresst wird, um günstige Druckverhältnisse zu erreichen.

Gemäß der Erfindung wird der Bolzen durch einen zweiten Kanal, der zumindest an einer Stelle eine maximal 30 %, vorzugsweise 3 % bis 20 %, insbesondere 5 % bis 15 %, kleinere Querschnittsfläche als der erste Kanal aufweist, gepresst. Durch eine entsprechende maximale Begrenzung einer Querschnittsfläche des zweiten Kanals bzw. einer Verjüngung kann eine erforderliche Presskraft in moderaten Grenzen gehalten werden. Ein gewisser Mindestwert Absenkung eines Querschnittes bzw. eine Mindestverjüngung im Ausmaß von 3 %, insbesondere 5 %, ist hingegen zweckmäßig, um gewünschte Druckverhältnisse sicherzustellen.

Das erfindungsgemäße Verfahren lässt sich bei beliebigen Winkeln zwischen dem ersten Kanal und dem zweiten Kanal anwenden, bevorzugt ist jedoch, dass ein Presswerkzeug mit einem Winkel von 60° bis 150°, insbesondere etwa 90° oder etwa 120°, zwischen dem ersten Kanal und dem zweiten Kanal eingesetzt wird.

Zweckmäßig ist es des Weiteren, dass der Bolzen einer Anzahl von Pressvorgängen unterworfen wird, die ein ganzzahliges Vielfaches von vier ist, und dabei vor jedem Pressvorgang um einen Winkel von 90° um eine Längsachse des Bolzens gedreht wird. Diese an sich bekannte Vorgehensweise stellt insbesondere bei runden Bolzen eine flankierende Maßnahme dar, um über den Querschnitt des Bolzens betrachtet ein homogenes, feines Gefüge zu erreichen.

Vorgesehen sein kann auch, dass der Bolzen mehrmals gepresst wird, wobei zumindest teilweise vor einem Pressvorgang der Bolzen umgedreht und ein hinteres Ende des Bolzens zuerst in das Presswerkzeug eingeführt wird. Dadurch kann eine Gefügehomogenität gesteigert werden, zumal an sich ein vorderes Ende des Bolzens einem geringeren Druck unterliegt, als ein im Bereich des Überganges der Kanäle befindlicher Abschnitt des Bolzens. Es wird dann sichergestellt, dass der Bolzen nach mehrmaligem Umformen ein zumindest weitgehend homogenes Gefüge aufweist.

Von Vorteil ist es, dass der Bolzen mehrmals gepresst wird, wobei eine Presstemperatur des Bolzens bei zumindest einem Pressvorgang gegenüber dem zuvor erfolgten Pressvorgang aktiv abgesenkt wird. Ein Pressen bei hoher Temperatur ist günstig, um Presskräfte möglichst gering zu halten und eine Rissbildung möglichst hintanzuhalten. Hingegen können bei tieferer Temperatur feinere Gefüge und bessere mechanische Kennwerte erreicht werden. Diesbezüglich hat sich als Optimum herausgestellt, zuerst einen ersten Pressvorgang bei einer höheren Temperatur zu beginnen und anschließend eine Presstemperatur bzw. Temperatur des Bolzens beim Pressen z. B. durch Kühlen abzusenken oder mit einem weiteren Pressvorgang zuzuwarten, bis der Bolzen und gegebenenfalls das Presswerkzeug eine vorgegebene niedrigere Temperatur erreicht hat. Beispielsweise kann bei einer Titanlegierung wie einer Ti64- oder Ti64ELI-Legierung eine erste Presstemperatur im Temperaturfenster von 500 °C bis 540 °C liegen, wonach die Presstemperatur bei jedem weiteren Pressvorgang um zumindest 5 °C, bevorzugt zumindest 10 °C, abgesenkt wird. Wird der Bolzen mehrmals gepresst, so liegt eine Temperatur beim letzten Pressvorgang vorzugsweise mehr als 50 °C unter der Temperatur beim ersten Pressvorgang. Mit dieser Maßnahme lässt sich in den ersten Pressschritten, die mit einer geringeren Presskraft durchgeführt werden können, bereits ein feines Gefüge einstellen. In daran anschließenden Pressvorgängen wird das Gefüge dann mit einer noch feineren Gefügestruktur ausgebildet. Bevorzugt ist in diesem Zusammenhang vorgesehen, dass die Presstemperatur bei jedem Pressvorgang abgesenkt wird, wenngleich je nach bearbeitetem Material auch andere Temperaturprogramme zum Einsatz kommen können. Beispielsweise kann eine Temperatur auch nur bei einem, vorzugsweise dem letzten, Pressvorgang abgesenkt sein, z. B. mehr als 50 °C im Vergleich mit einem ersten oder den vorhergehenden Pressvorgängen.

Eine Absenkung der Presstemperatur bei einzelnen Pressvorgängen ist insbesondere dann bevorzugt, wenn ein hexagonal kristallisierendes Metall oder eine dergleichen Legierung eingesetzt wird, da bei einem hexagonalen Kristallgitter eine entsprechende Verfahrensweise eine große Auswirkung auf eine Gefügehomogenität zeigt.

Vorteilhaft kann es auch sein, dass der gepresste Bolzen vor oder zur Erstellung des Gegenstandes einem isothermen Schmieden unterworfen wird. Unter einem isothermen Schmieden wird in diesem Zusammenhang ein Schmieden verstanden, bei dem das Schmiedewerkzeug und der gepresste Bolzen im Wesentlichen dieselbe Temperatur aufweisen. Bei einem isothermen Schmieden ist insbesondere von Vorteil, dass nicht nur die durch ECAP-Umformung erzielte Mikrostruktur bzw. das feine Gefüge mit den hervorragenden Eigenschaften erhalten bleibt, sondern auch die mechanischen Kennwerte noch weiter gesteigert werden können. Eine statische Rekristallisation tritt bei einem isothermen Schmieden nicht auf.

Grundsätzlich ist es zweckmäßig, dass der Bolzen vor dem Pressvorgang erwärmt wird, damit sich dieser leichter pressen lässt. Eine Erwärmung kann bei einer geringen Anzahl von zu pressenden Bolzen direkt im Presswerkzeug durchgeführt werden, wenn dieses beispielsweise mit Heizpatronen bestückt ist. Vor allem bei einer größeren Anzahl von zu pressenden Bolzen kann vorgesehen sein, dass der Bolzen außerhalb des Presswerkzeuges erwärmt wird, beispielsweise in einem Ofen oder durch induktives Heizen. Es können dann mehrere Bolzen stets für den Pressvorgang bereitgehalten werden und eine Durchsatzmenge ist lediglich durch eine Taktzeit des Presswerkzeuges limitiert.

Mit einem erfindungsgemäßen Verfahren können beliebige Gegenstände hergestellt werden, beispielsweise Bauteile für die Luft- und Raumfahrt. Bevorzugt ist jedoch eine Anwendung des erfindungsgemäßen Verfahrens zur Herstellung von Gegenständen, die im oder am menschlichen Körper zu medizinischen Zwecken eingesetzt werden, insbesondere Implantate, z. B. Hüftimplantate, Marknägel oder Knochenplatten.

Die weitere Aufgabe der Erfindung wird erfindungsgemäß durch ein Presswerkzeug gelöst, wie dieses im Anspruch 13 definiert ist.

Mit einem erfindungsgemäßen Presswerkzeug können Bolzen mit guten mechanischen Eigenschaften und hoher Homogenität ohne großen apparativen Aufwand erstellt werden. Durch die an zumindest einer Stelle vorgesehene Durchmesserverringerung erfährt ein Bolzen beim Pressen einen hydrostatischen Druck bzw. eine Art Gegendruck, was günstige Umformbedingungen ergibt. Der apparative Aufwand hierfür ist minimal, da im Gegensatz zum Stand der Technik keine zusätzlichen Komponenten für ein Erzeugen eines Gegendrucks erforderlich sind.

Der zweite Kanal kann beispielsweise von einer ersten Stelle in Richtung zu einem Auslass hin zumindest teilweise verjüngt, vorzugsweise konisch verjüngt, ausgebildet sein. Möglich ist es, dass bereits im ersten Drittel des zweiten Kanals eine Verjüngung beginnt, die sich bis zum Auslass des zweiten Kanals erstreckt. Möglich ist es aber ebenso, dass der zweite Kanal vorerst einen gleichen Durchmesser aufweist wie der erste Kanal, dann aber im genannten ersten Drittel eine Stufe vorgesehen ist, an welcher sich ein freier Durchmesser des zweiten Kanals verkleinert. Die Stufe ist in diesem Fall ebenfalls konisch in Richtung zum Auslass hin ausgebildet, jedoch wesentlich kürzer als eine über weite Bereiche des zweiten Kanals bis hin zum Auslass verlaufende konische Verjüngung. Im Anschluss an die Stufe bzw. Verjüngung kann der zweite Kanal dann mit einem konstanten freien Durchmesser ausgebildet sein.

Aus den bereits erläuterten Gründen ist vorgesehen, dass der zweite Kanal an zumindest einer Stelle eine 3 % bis maximal 30 %, vorzugsweise 3 % bis 20 %, insbesondere 5 % bis 15 %, kleinere Querschnittsfläche als der erste Kanal aufweist.

Weitere Merkmale, Vorteile und Wirkungen der Erfindung ergeben sich aus dem nachfolgend dargestellten Ausführungsbeispiel. In den Zeichnungen, auf welche dabei Bezug genommen wird, zeigen:
Fig. 1 ein Presswerkzeug;
Fig. 2 mechanische Kennwerte einer Legierung im Ausgangszustand sowie nach Umformung mittels verschiedener Verfahren;
Fig. 3 eine Fotografie von Zellen auf einem Bolzen;
Fig. 4 eine Fotografie von Zellen auf einem Bolzen gemäß Fig. 3, wobei der Bolzen einer ECAP-Umformung unterworfen wurde;
Fig. 5 ein Diagramm betreffend eine Biokompatibilität einer Ti64ELI-Legierung nach einer ECAP-Umformung;
Fig. 6 ein Diagramm betreffend eine Hämokompatibilität einer Ti64ELI-Legierung nach einer ECAP-Umformung.

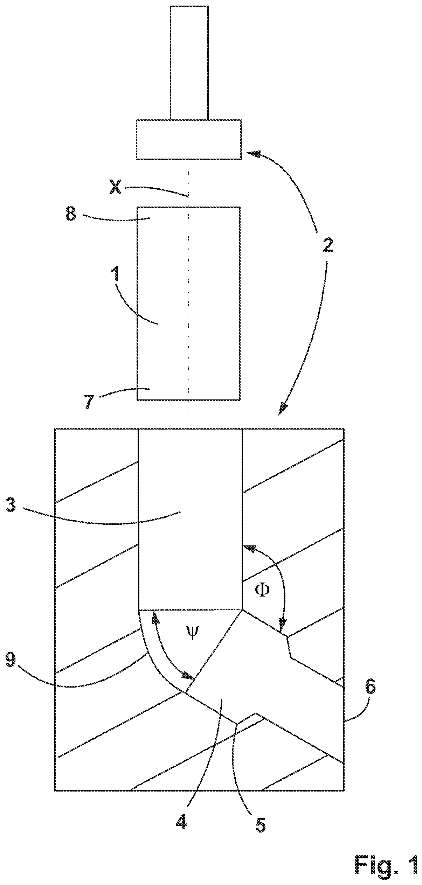

In Fig. 1 ist ein erfindungsgemäßes Presswerkzeug 2 sowie ein in diesem zu pressender runder Bolzen 1 mit einer Längsachse X im Querschnitt dargestellt. Das Presswerkzeug 2 weist einen ersten Kanal 3 und einen daran in einem Winkel Φ von etwa 120° anschließenden zweiten Kanal 4 auf. Der erste Kanal 3 ist im Querschnitt rund ausgebildet. Der zweite Kanal 4 schließt an den ersten Kanal 3 einerseits über eine Ecke und andererseits über eine Rundung 9, die einen Winkel Ψ von etwa 55° überstreicht, an und ist im Anschlussbereich mit einem gleichen Querschnitt und einem gleichen freien Durchmesser wie der erste Kanal 3 ausgebildet. Im Unterschied zum ersten Kanal 3 ist jedoch im zweiten Kanal 4 eine erste Stelle 5 vorgesehen, an welcher der zweite Kanal 4 eine zu einem Auslass 6 hin zulaufende abgeschrägte konische Fläche aufweist, sodass sich ein freier Durchmesser des zweiten Kanals 4 verkleinert. Die Verjüngung kann wie in Fig. 1 dargestellt stufenförmig sein und nach einer Abschrägung ab einer zweiten Stelle wieder parallel zu einem Bereich vor der Abschrägung verlaufen. Möglich ist es aber auch, dass der zweite Kanal 4 von der ersten Stelle 5 bis zum Auslass 6 hin kontinuierlich verjüngt ausgebildet ist. Unabhängig von der konkreten Ausbildung ist es zweckmäßig, dass die erste Stelle 5 bereits in einem ersten Drittel der Länge des zweiten Kanals 4 von der Rundung 9 zum Auslass 6 hin betrachtet angeordnet ist bzw. eine kontinuierliche konische Verjüngung bereits in diesem Bereich beginnt. Dadurch wird erreicht, dass ein vorderes Ende 7 des Bolzens 1 bei jedem Pressvorgang auf die erste Stelle 5 bzw. die Verjüngung trifft, sodass dem Bolzen 1 ein Widerstand entgegengesetzt wird, wodurch sich beim Umformen ein Gegendruck aufbaut.

Mit einem Presswerkzeug 2 wurde ein Bolzen 1 aus einer Ti64-Legierung mit 6 Masse-% Aluminium und 4 Masse-% Vanadium umgeformt. Bei der Legierung handelte es sich im Konkreten um eine im Handel erhältliche Legierung mit der Bezeichnung "Titanium Ti-6Al-4V extra low interstitial" (3.7165 ELI, ISO 5832-2; ASTM F 136) mit maximal 0,13 Masse-% Sauerstoff, maximal 0,015 Masse-% Wasserstoff, maximal 0,08 Masse-% Kohlenstoff, maximal 0,20 Masse-% Eisen, maximal 0,03 Masse-% Stickstoff und einem Gehalt von 5,5 bis 6,5 Masse-% Aluminium sowie 3,5 bis 4,5 Masse-% Vanadium, maximal 0,4 Masse-% Verunreinigungen und Titan als Rest. Ein Bolzen 1 aus einer derartigen Legierung wurde achtmal mit dem Presswerkzeug 2 gepresst, wobei der Bolzen 1 nach jeder Umformung 90° um die Längsachse X gedreht wurde. Eine Temperatur des Bolzens 1 bei den einzelnen Pressvorgängen wurde so eingestellt, dass der Bolzen 1 erstmalig bei 530 °C und anschließend jeweils mit einer Temperatur von 10 °C weniger gepresst wurde. Anschließend wurde der so gepresste Bolzen 1 in Bezug auf mechanische Kennwerte sowie eine potenzielle Eignung als Halbzeug zur Herstellung eines Implantats durch Schmieden oder spanabhebende Bearbeitung untersucht. Darüber hinaus wurde ein analog umgeformter Bolzen 1 einem isothermen Schmieden bei 550 °C unterworfen.

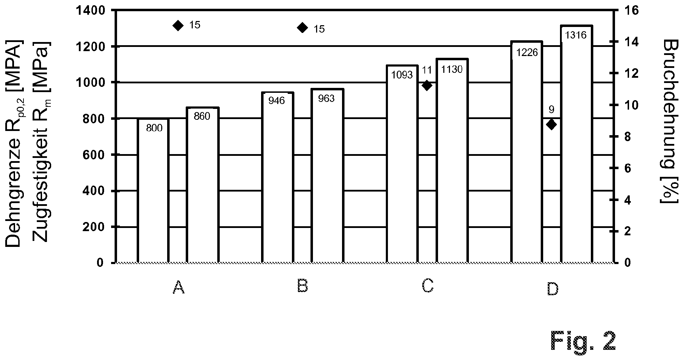

In Fig. 2 sind mechanische Kennwerte eines Bolzens 1 in einem Zustand C nach einer Umformung in der vorstehend beschriebenen Weise im Vergleich mit einem Ausgangszustand A und einem Zustand B einer konventionellen Umformung (Schmieden bei 890 °C) dargestellt. Des Weiteren sind mechanische Kennwerte für einen Zustand D dargestellt, die sich auf einen Bolzen gemäß Zustand C, jedoch mit nachfolgendem isothermen Schmieden bei 550 °C beziehen. Wie ersichtlich ist, ist eine Dehngrenze und eine Zugfestigkeit für den Zustand C deutlich höher als für den Ausgangszustand A sowie den Zustand B einer konventionellen Verarbeitung. Des Weiteren ist aus den Daten zum Zustand D ersichtlich, dass ein isothermes Schmieden eines zuvor massiv umgeformten Bolzens 1 zu einer zusätzlichen Erhöhung der Festigkeitswerte führt.

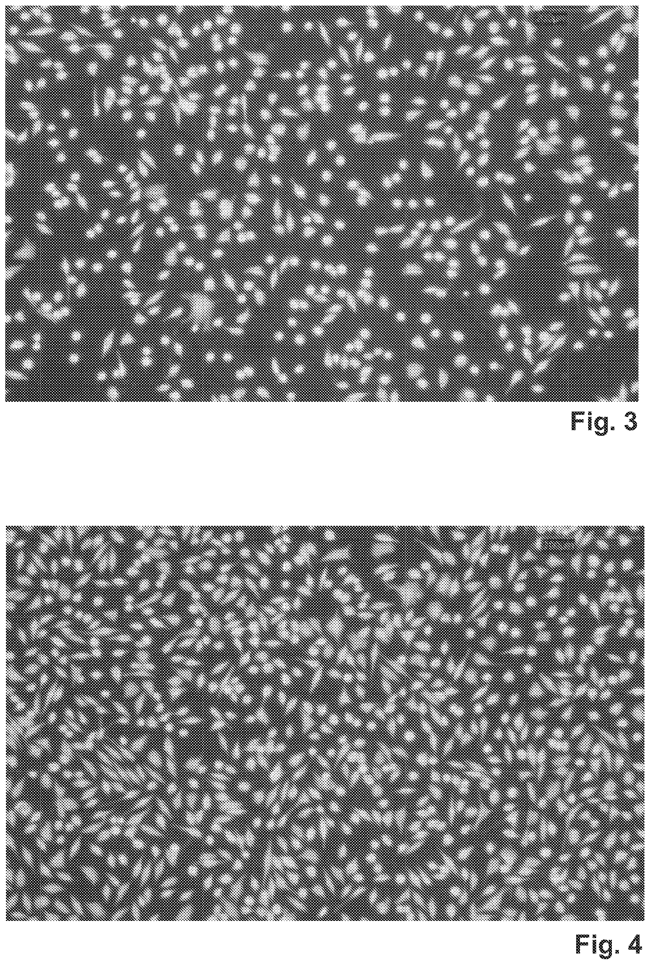

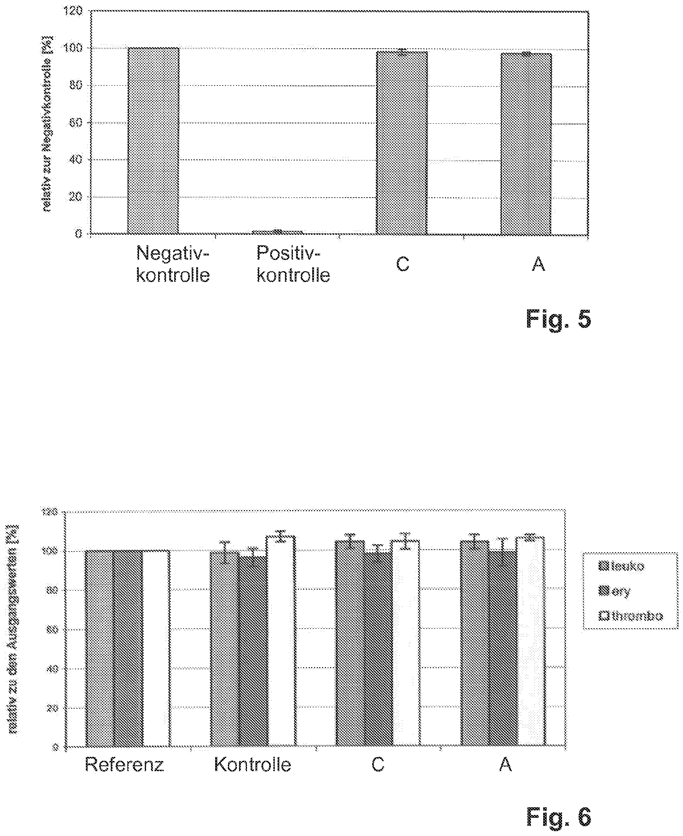

In Fig. 3 ist eine Fotografie gezeigt, in der L929-Zellen nach einer 24-stündigen Inkubation auf einem Ausgangsmaterial gemäß Ausgangszustand A ersichtlich sind. Fig. 4 zeigt ein entsprechendes Foto für einen Bolzen 1, der wie beschrieben massiv umgeformt wurde und den Zustand C aufweist. Wie sich aus einem Vergleich ergibt, sind auf dem Bolzen 1 gemäß Zustand C, also nach mehrmaliger massiver Umformung, deutlich mehr Zellen nach der Inkubation vorhanden, was auf eine exzellente Eignung des Bolzens 1 zur Herstellung von biokompatiblen Erzeugnissen, insbesondere Implantaten, schließen lässt. Dies wird in Fig. 5 bestätigt, wonach eine Mitochondrienaktivität nach 24-stündiger Inkubation gleich hoch ist wie für einen Ausgangszustand A bzw. annähernd die Zytotoxizitätswerte einer Negativkontrolle erreicht. Ebenso ist gemäß Fig. 6 eine Hämokompatibilität gegeben, da die Zellzahlen von Leuko-, Ery- und Thrombozyten in einem Differenzblutbild auf einem Referenzwertniveau liegen. Im Übrigen konnten in wässrigen Extrakten eines Bolzens 1 weder Aluminium- noch Vanadiumionen nachgewiesen werden.

## Patentansprüche

1. Verfahren zur Herstellung eines Gegenstandes aus einem Metall oder einer Legierung, insbesondere einer Titanlegierung, wobei in einem Pressvorgang ein Bolzen (1) gegebenenfalls mehrmals wiederholt durch einen ersten Kanal (3) und einen daran anschließenden zweiten Kanal (4) eines Presswerkzeuges (2) gepresst wird, wonach aus dem gepressten Bolzen (1) der Gegenstand wie ein Implantat erstellt wird, **dadurch gekennzeichnet, dass** der Bolzen (1) durch einen im Querschnitt zumindest an einer Stelle einen kleineren Durchmesser als der erste Kanal (3) aufweisenden zweiten Kanal (4) gepresst wird, wobei der erste Kanal (3) und der zweite Kanal (4) einen von 0° verschiedenen Winkel (Φ) miteinander einschließen, und wobei der zweite Kanal (4) an den ersten Kanal (3) mit gleichem freien Durchmesser und gleichem Querschnitt anschließt und eine Verjüngung mit einer 3 % bis maximal 30 % kleineren Querschnittsfläche als der erste Kanal (3) aufweist.

2. Verfahren nach Anspruch 1, **dadurch gekennzeichnet, dass** der Bolzen (1) durch einen von einer ersten Stelle (5) in Richtung zu einem Auslass (6) hin zumindest teilweise verjüngten, vorzugsweise konisch verjüngten, zweiten Kanal (4) gepresst wird.

3. Verfahren nach Anspruch 1 oder 2, **dadurch gekennzeichnet, dass** der Bolzen (1) durch einen zweiten Kanal (4), der zumindest an einer Stelle eine 3 % bis 20 %, insbesondere 5 % bis 15 %, kleinere Querschnittsfläche als der erste Kanal (3) aufweist, gepresst wird.

4. Verfahren nach einem der Ansprüche 1 bis 3, **dadurch gekennzeichnet, dass** ein Presswerkzeug (2) mit einem Winkel (Φ) von 60° bis 150°, insbesondere etwa 90° oder etwa 120°, zwischen dem ersten Kanal (3) und dem zweiten Kanal (4) eingesetzt wird.

5. Verfahren nach einem der Ansprüche 1 bis 4, **dadurch gekennzeichnet, dass** der Bolzen (1) einer Anzahl von Pressvorgängen unterworfen wird, die ein ganzzahliges Vielfaches von vier ist, und dabei vor jedem Pressvorgang um einen Winkel von 90° um eine Längsachse (X) des Bolzens (1) gedreht wird.

6. Verfahren nach einem der Ansprüche 1 bis 5, **dadurch gekennzeichnet, dass** der Bolzen (1) mehrmals gepresst wird, wobei zumindest teilweise vor einem Pressvorgang der Bolzen (1) umgedreht und ein hinteres Ende (8) des Bolzens (1) zuerst in das Presswerkzeug (2) eingeführt wird.

7. Verfahren nach einem der Ansprüche 1 bis 6, **dadurch gekennzeichnet, dass** der Bolzen (1) mehrmals gepresst wird, wobei eine Presstemperatur des Bolzens (1) bei zumindest einem Pressvorgang gegenüber dem zuvor erfolgten Pressvorgang aktiv abgesenkt wird.

8. Verfahren nach Anspruch 7, **dadurch gekennzeichnet, dass** die Presstemperatur bei jedem Pressvorgang abgesenkt wird.

9. Verfahren nach Anspruch 7 oder 8, **dadurch gekennzeichnet, dass** ein hexagonal kristallisierendes Metall oder eine dergleichen Legierung eingesetzt wird.

10. Verfahren nach einem der Ansprüche 1 bis 9, **dadurch gekennzeichnet, dass** der gepresste Bolzen (1) vor oder zur Erstellung des Gegenstandes einem isothermen Schmieden unterworfen wird.

11. Verfahren nach einem der Ansprüche 1 bis 10, **dadurch gekennzeichnet, dass** der Bolzen (1) vor dem Pressvorgang erwärmt wird.

12. Verfahren nach Anspruch 11, **dadurch gekennzeichnet, dass** der Bolzen (1) außerhalb des Presswerkzeuges (2) erwärmt wird.

13. Presswerkzeug (2) zum Umformen eines Bolzens (1), aufweisend einen ersten Kanal (3) und einen daran anschließenden zweiten Kanal (4), wobei der erste Kanal (3) und der zweite Kanal (4) einen von 0° verschiedenen Winkel (Φ) miteinander einschließen, **dadurch gekennzeichnet, dass** der zweite Kanal (4) zumindest an einer Stelle einen kleineren Durchmesser als der erste Kanal (3) aufweist, wobei der zweite Kanal (4) an den ersten Kanal (3) mit gleichem freien Durchmesser und gleichem Querschnitt anschließt und eine Verjüngung mit einer 3 % bis maximal 30 % kleineren Querschnittsfläche als der erste Kanal (3) aufweist.

14. Presswerkzeug (2) nach Anspruch 13, **dadurch gekennzeichnet, dass** der zweite Kanal (4) von einer ersten Stelle (5) in Richtung zu einem Auslass (6) hin zumindest teilweise verjüngt, vorzugsweise konisch verjüngt, ist.

15. Presswerkzeug (2) nach Anspruch 13 oder 14, **dadurch gekennzeichnet, dass** der zweite Kanal (4) an zumindest einer Stelle eine 3 % bis 20 %, insbesondere 5 % bis 15 %, kleinere Querschnittsfläche als der erste Kanal (3) aufweist.

## Claims

1. A method for the manufacture of an article produced from a metal or an alloy, in particular from a titanium alloy, wherein, in a pressing procedure which is optionally repeated a plurality of times, a pin (1) is pressed through a first channel (3) and through a second channel (4) of a pressing tool (2) which is contiguous therewith, whereupon the article such as an implant is produced from the pressed pin (1), **characterized in that** the pin (1) is pressed through a second channel (4) which has a cross section that has a smaller diameter than the first channel (3) at one position at least, wherein the first channel (3) and the second channel (4) enclose an angle (φ) with respect to each other which differs from 0°, and wherein the second channel (4) communicates with the first channel (3) with the same free diameter and the same cross section and has a taper with a 3% to a maximum of 30% smaller cross sectional area than the first channel (3).

2. The method as claimed in claim 1, **characterized in that** the pin (1) is pressed through a second channel (4) which tapers at least partially, preferably tapers conically, from a first position (5) in the direction towards an outlet (6).

3. The method as claimed in claim 1 or claim 2, **characterized in that** the pin (1) is pressed through a second channel (4) which has, at one position at least, a 3% to 20%, in particular 5% to 15% smaller cross-sectional area than the first channel (3).

4. The method as claimed in one of claims 1 to 3, **characterized in that** a pressing tool (2) is used with an angle (φ) of 60° to 150°, in particular approximately 90° or approximately 120°, between the first channel (3) and the second channel (4).

5. The method as claimed in one of claims 1 to 4, **characterized in that** the pin (1) undergoes a number of pressing procedures which is a whole number multiple of four, and wherein prior to each pressing procedure, it is turned through an angle of 90° about a longitudinal axis (X) of the pin (1).

6. The method as claimed in one of claims 1 to 5, **characterized in that** the pin (1) is pressed a plurality of times wherein, prior to a pressing procedure, the pin (1) is turned at least partially and a back end (8) of the pin (1) is introduced into the pressing tool (2) first.

7. The method as claimed in one of claims 1 to 6, **characterized in that** the pin (1) is pressed a plurality of times, wherein during at least one pressing procedure, a pressing temperature of the pin (1) is actively reduced compared with the previously completed pressing procedure.

8. The method as claimed in claim 7, **characterized in that** the pressing temperature is reduced for each pressing procedure.

9. The method as claimed in claim 7 or claim 8, **characterized in that** a metal or like alloy which crystallizes in the hexagonal system is employed.

10. The method as claimed in one of claims 1 to 9, **characterized in that** the pressed pin (1) undergoes isothermal forging prior to or in order to produce the article.

11. The method as claimed in one of claims 1 to 10, **characterized in that** the pin (1) is heated prior to the pressing procedure.

12. The method as claimed in claim 11, **characterized in that** the pin (1) is heated outside the pressing tool (2).

13. A pressing tool (2) for shaping a pin (1), comprising a first channel (3) and a second channel (4) which is contiguous therewith, wherein the first channel (3) and the second channel (4) enclose an angle (φ) which differs from 0°, **characterized in that** at one position at least, the second channel (4) has a smaller diameter than the first channel (3), wherein the second channel (4) communicates with the first channel (3) with the same free diameter and the same cross section and has a taper with a 3% to a maximum of 30% smaller cross-sectional area than the first channel (3).

14. The pressing tool (2) as claimed in claim 13, **characterized in that** the second channel (4) tapers at least partially, preferably tapers conically, from a first position (5) in the direction towards an outlet (6).

15. The pressing tool (2) as claimed in claim 13 or claim 14, **characterized in that** the second channel (4) has, at one position at least, a 3% to 20%, in particular 5% to 15% smaller cross-sectional area than the first channel (3).

## Revendications

1. Procédé de fabrication d'un objet à partir d'un métal ou d'un alliage, en particulier d'un alliage de titane, dans lequel, dans un processus de presse, une broche (1) est éventuellement pressée plusieurs fois à répétition à travers un premier canal (3) et un second canal s'y raccordant (4) d'un outil de presse (2), après quoi l'objet est réalisé comme un implant à partir de la broche pressée (1), **caractérisé en ce que** la broche (1) est pressée à travers un second canal (4) présentant en section transversale, du moins à un endroit, un diamètre inférieur à celui du premier canal (3), le premier canal (3) et le second canal (4) circonscrivant ensemble un angle (φ) différent de 0° et le second canal (4) se raccordant au premier canal (3) de même diamètre libre et de même section transversale et présentant un rétrécissement ayant une surface de section transversale inférieure de 3 % à 30 % au maximum à celle du premier canal (3).

2. Procédé selon la revendication 1, **caractérisé en ce que** la broche (1) est pressée à travers un second canal (4) au moins partiellement rétréci, de préférence rétréci en forme conique, depuis un premier point (5) en direction d'une sortie (6).

3. Procédé selon la revendication 1 ou 2, **caractérisé en ce que** la broche (1) est pressée à travers le second canal (4) qui présente, du moins à un endroit, une surface de section transversale inférieure de 3 % à 20 %, en particulier de 5 % à 15 %, à celle du premier canal (3).

4. Procédé selon une des revendications 1 à 3, **caractérisé en ce qu'**on utilise un outil de presse (2) avec un angle (φ) de 60° à 150°, en particulier environ 90° ou environ 120°, entre le premier canal (3) et le second canal (4).

5. Procédé selon une des revendications 1 à 4, **caractérisé en ce que** la broche (1) est soumise à un nombre de processus de presse qui est un multiple entier de quatre et est alors tournée avant chaque processus de presse suivant un angle de 90° autour d'un axe longitudinal (X) de la broche (1).

6. Procédé selon une des revendications 1 à 5, **caractérisé en ce que** la broche (1) est pressée plusieurs fois, la broche (1) étant du moins partiellement retournée avant un processus de presse et une extrémité arrière (8) de la broche (1) étant d'abord introduite dans l'outil de presse (2).

7. Procédé selon une des revendications 1 à 6, **caractérisé en ce que** la broche (1) est pressée plusieurs fois, une température de presse de la broche (1) étant abaissée activement pendant au moins un processus de presse par rapport au processus de presse ayant eu lieu précédemment.

8. Procédé selon la revendication 7, **caractérisé en ce que** la température de presse est diminuée lors de chaque processus de presse.

9. Procédé selon la revendication 7 ou 8, **caractérisé en ce qu'**on utilise un métal cristallisant en hexagone ou un alliage similaire.

10. Procédé selon une des revendications 1 à 9, **caractérisé en ce que** la broche pressée (1) est soumise à un forgeage isotherme avant ou pour la réalisation de l'objet.

11. Procédé selon une des revendications 1 à 10, **caractérisé en ce que** la broche (1) est chauffée avant le processus de presse.

12. Procédé selon la revendication 11, **caractérisé en ce que** la broche (1) est chauffée en dehors de l'outil de presse (2).

13. Outil de presse (2) pour le formage d'une broche (1), présentant un premier canal (3) et un second canal s'y raccordant (4), le premier canal (3) et le second canal (4) circonscrivant ensemble un angle (φ) différent de 0°, **caractérisé en ce que** le second canal (4) présente à un endroit un diamètre inférieur à celui du premier canal (3), le second canal (4) se raccordant au premier canal (3) de même diamètre libre et de même section transversale et présentant un rétrécissement ayant une surface de section transversale inférieure de 3 % à 30 % au maximum à celle du premier canal (3).

14. Outil de presse (2) selon la revendication 13, **caractérisé en ce que** le second canal (4) se rétrécit du moins partiellement, de préférence en forme conique, depuis un premier point (5) en direction d'une sortie (6).

15. Outil de presse (2) selon la revendication 13 ou 14, **caractérisé en ce que** le second canal (4) présente à au moins un endroit une surface de section transversale inférieure de 3 % à 20 %, en particulier de 5 % à 15 %, à celle du premier canal (3).
